# EUROPEAN PATENT APPLICATION

(11) **EP 1 044 691 A2**
(43) Date of publication of application: **18.10.2000**
(21) Application number: 00250026.2
(22) Date of filing: 27.01.2000
(51) Int. Cl.: A61K 47/48

(54) **Use of glycosaminoglycan lipid conjugates for inducing osteogenesis**

(30) Priority: 28.01.1999 JP 1971299
(71) Applicant: Tokyo Medical and Dental University, Tokyo (JP)
(72) Inventor: Kawauchi, Toshiyuki, Yokohama City, Kanagawa Pref. (JP); Takahashi, Makoto, Tokyo (JP); Shinomiya, Kenichi, Tokyo (JP)
(74) Representative: Wablat, Wolfgang, Dr.Dr.

(57) **Abstract**

A novel medicine capable of inducing osteogenesis at lower dosage is provided. The osteogenesis inducing medicine contains a lipid-bound glycosaminoglycan, composed of a glycosaminoglycan conjugated with a lipid, or a pharmacologycally accepted salt of the lipid-bound glycosaminoglycan as the effective ingredient of the medicine.

## Description

### BACKGROUND OF THE INVENTION

### 1. Field of the invention

This invention relates to a medicine for inducing osteogenesis.

### 2. Description of Related Art

In the field of medical treatment, particularly in the field of plastic surgery, there has been a strong demand on a medicine or a medical device which is capable of inducing osteogenesis.

Glycosaminoglycans have been known to exhibit osteogenesis inducing activity. For example, Japanese patent laid-open publication "kokai" 508973/1996 discloses osteogenesis induction activity of hyaluronic acid with a molecular weight of 20,000 to 60,000, at a concentration of 0.5 mg/ml. Moreover, Japanese patent laid-open publication "kokai" 64367/1987 discloses a material for artificial bone, composed of a raw material such as glycosaminoglycan, collagen or apatite. Moreover, it discloses enhancement of osteogenesis achieved by said material.

Glycosaminoglycan diffuses in a living body rapidly, because of its high solubility in water. Therefore, it was difficult to achieve a certain concentration of glycosaminoglycan sufficient to induce osteogenesis in the target organ and retain the concentration for a sustained period.

Therefore, the purpose of this invention is to provide a novel pharmaceutical medicine which can achieve induction of osteogenesis efficiently at smaller administration doses or lower concentrations of the medicine in the target organ.

Some lipid-bound glycosaminoglycans were disclosed in Japanese patent laid-open publications "kokai" 80201/1992, 80202/1992 and 30979/1997.

The inventors paid attention on said lipid-bound glycosaminoglycans and found their activity to induce osteogenesis. The lipid-bound glycosaminoglycan exhibited considerable osteogenesis induction activity. Surprisingly, the lipid-bound glycosaminoglycan induced osteogenesis even at 1/100 of a concentration necessary for the induction when glycosaminoglycan without lipid is administrated. This invention was achieved according to the findings.

Therefore, this invention provides a medicine for inducing osteogenesis, which contains a lipid-bound glycosaminoglycan composed of a glycosaminoglycan conjugated with a lipid, or a pharmacologycally accepted salt of the lipid-bound glycosaminoglycan, as the effective ingredient of the medicine.

Glycosaminoglycan contained in the osteogenesis inducing medicine of this invention may preferably be hyaluronic acid, chondroitin, chondroitin sulfate, chondroitin polysulfate, dermatan sulfate, heparin, keratan sulfate or keratan polysulfate. Moreover, the lipid contained in the osteogenesis inducing medicine of this invention may preferably be a glycerolipid and the glycerolipid may preferably be a phospholipid and said phospholipid may preferably be phosphatidylethanolamine. In the more preferred embodiments, the glycosaminoglycan is hyaluronic acid and said lipid is phosphatidylethanolamine. The lipid may preferably be conjugated to the reduced terminal of glycosaminoglycan by covalent bonding.

The lipid-bound glycosaminoglycans have been already known as described above. Though, applicability of the compound as an anti-metastasis medicine, an anti-rheumatoid medicine and an anti-neuromatic disease medicine have been disclosed in Japanese patent laid-open publications "kokai" 82836/1992, 72893/1994 and 30979/1997, respectively. Therefore, they have not disclosed nor suggested applicability of a lipid-bound glycosaminoglycan as an osteogenesis inducing medicine.

Moreover, the previous techniques concerning induction of osteogenesis activity described above or assisting of osteoplasty are as follows. Japanese patent laid-open publication "kokai" 508973/1996 discloses a technique utilizing hyarulonic acid and Japanese patent laid-open publication "kokai" 64367/1987 discloses an artificial bone utilizing condroitin-4-sulfate.

Therefore, these techniques are using the glycosaminoglycans themselves and usage of glycosaminoglycan derivatives have not disclosed nor suggested.

This invention will be described in detail by following description and drawings.

### BRIEF DESCRIPTION OF THE DRAWINGS

Fig. 1 shows autoradiographies of northern blotting, detecting expression of ALP, COL1 and OC genes induced by HA-PE; and
Fig.2 shows graphs obtained by digitalization of northern blotting shown in Fig. 1.

### DETAILED DESCRIPTION OF EMBODIMENTS

The osteogenesis inducing medicine of this invention contains a lipid-bound glycosaminoglycan wherein a lipid is conjugated to a glycosaminoglycan or its pharmaceutical accepted salts, as the effective ingredient.

The lipid-bound glycosaminoglycan of this invention contains a backbone of glycosaminoglycan, which is a polysaccharide composed of a repeated unit of a disaccharide. The repeated unit is composed of D-amino sugar such as glucosamine or D-galactosamine and uronic acid such as D-glucuronic acid, L-iduronic acid or D-galacturonic acid. The glycosaminoglycan may be either of an extracted natural substance that originated from an animal or a cultivated substance originated from a micro-organism. It can also be a chemically or enzymatically synthesized substance. For example, the glycosaminoglycan may be hyaluronic acid, condroitin, chondroitin sulfates (chondroitin sulfate A, chondroitin sulfate C, chondroitin sulfate E, chondroitin sulfate K), chondroitin polysulfate, dermatan sulfate, heparin, keratan sulfate or keratan polysulfate. But the examples described are not to limit the range of glycosaminoglycans. The glycosaminoglycan can be its pharmaceutical accepted salts of conventional use.

Hyaluronic acid is particularly preferred for the glycosaminoglycan. The molecular weight of hyaluronic acid may be from several thousands to 20,000,000, preferably from 15,000 to 1,000,000, more preferably from 20,000 to 800,000.

Moreover, a compound lipid or a simple lipid can be conjugated to the glycosaminoglycan described above. The lipid can be a naturally occurred substance derived from an animal, a plant or a micro-organism. It can be a synthesized or partially degraded substance by a chemical or enzymatical technique. The examples of lipids available are glycerolipids such as phospholipids, fatty-acids with long-chain, fatty acid amines with long-chain, cholesterols, sphingolipids and ceramids. In particular, phospholipids such as phosphatidyl-ethanolamine, phosphatidyl-serine, phosphatidyl-threonine, ethanolamine-plasmalogen, serine-plasmalogen, lysophosphatidyl-choline or lysophosphatidyl-inositol are preferred. Moreover, glycerolipids of neutral lipids such as monoacylglycerol or diacylglycerol are also preferred. Phospholipids containing a primary amino group are particularly preferred. The acyl group of the lipids may have any length of carbon chain and any extent of desaturation, but lipids with 6 carbons or above are preferred. Palmitoyl (hexadecanoyl) or stearoyl (octadecanoyl) group may be preferable as the acyl group. These lipids can also be their pharmaceutical accepted salts of conventional use.

The binding site of the glycosaminoglycan and the lipid is not limited. For the binding site, the terminal portion of glycosaminoglycan is preferred and the reduced terminal portion of glycosaminoglycan is particularly preferred. Moreover, a chemical bonding is preferred bonding form and covalent bonding may be the most preferred bonding form.

For the binding site of covalent bonding consisting the lipid-bound glycosaminoglycan, following groups of glycosaminoglycan or lipid may be preferred. Preferred binding site of a glycosaminoglycan includes but not limited to carboxyl group (containing lactone), formyl group (containing hemiacetal group), hydroxyl group or primary amino group, or said groups additionally incorporated into the glycosaminoglycan. Preferred binding site of a lipid includes but not limited to carboxyl group, formyl group, primary amino group or said groups additionally incorporated into the lipid. Preferred bonding form includes but not limited to peptide bonding (-CO-NH-), ester bonding or amino-alkyl bonding (-CH₂-NH-) formed between said group of the glycosaminoglycan and said group of the lipid.

Especially, followings are the examples of preferred forms of bonding.
(1) A peptide bonding (-CO-NH-) formed between carboxyl group (containing lactone) of a glycosaminoglycan and primary amino group of a lipid is preferable. The carboxyl group is formed by the cleavage of the pyranose ring existing at the reduced terminal of the glycosaminoglycan.
(2) A peptide bonding (-CO-NH-) formed between carboxyl group of uronic acid existing in a glycosaminoglycan and primary amino group of a lipid is preferable.
(3) An amino-alkyl bonding (-CH₂-NH-) formed by reduction of a sniff base is preferable. The shiff base is formed by the reaction of formyl group of a glycosaminoglycan and primary amino group of a lipid. The formyl group of glycosaminoglycan is formed by the cleavage of the pyranose ring existing at its reduced terminal by chemical treatment.

Then, the amino group, the carboxyl group, the formyl group (containing hemiacetal group) and the hydroxyl group involving chemical bonding described above may be the groups existing in the glycosaminoglycan or in the lipid before chemical treatment. Alternatively, the groups involving chemical bonding may be formed by chemical treatment of the glycosaminoglycan or the lipid. The groups may be formed by additional incorporation of a spacer compound, containing said group at its terminal, into the glycosaminoglycan or the lipid.

In the lipid-bound glycosaminoglycan, the lipid may be more preferably conjugated to the reduced terminal of glycosaminoglycan by covalent bonding. The methods to produce such compounds are disclosed in Japanese patent laid-open publications "kokai" 80201/1992, 80202/1992 and 30979/1997. For example, the methods described below can be utilized to produce the lipid-bound glycosaminoglycan.

### Restricted oxidation of reduced terminal of glycosaminoglycan

According to this method, galactose residue, uronic acid residue or hexosamine residue are exposed to restricted oxidation (partial oxidation). These residues are sugar residues of the reduced terminal of a glycosaminoglycan. As the result of partial oxidation, the pyranose ring existing at the reduced terminal of glycosaminoglycan is opened (cleaved) specifically. Moreover, an aldehyde compound is produced by formation of formyl group at the reduced terminal of glycosaminoglycan. Then reaction between the formyl group of the aldehyde compound and the primary amino group of the lipid is performed to produce a shift base. The covalent aminoalkyl bonding (-CH₂-NH-), between the glycosaminoglycan and the lipid, is formed by the reduction of said shiff base.

The reduction of sugar residue at the reduced terminal of glycosaminoglycan can be achieved by treatment using 5 to 50 equivalents, preferably 25 to 30 equivalents, of reductant. The reductant may be alkaline salts of boron hydrides, such as sodium boron hydride, sodium cyano boron hydride dissolved in some adequate aqueous solvent (for example, water or borate salt buffer). The reaction may be performed usually under 10 to 30°C, preferably under 15 to 25°C.

After reducing reaction described above, restricted oxidation is performed to produce an aldehyde compound, containing formyl group at the reduced terminal of glycosaminoglycan. Restricted oxidation may be performed using 1 to 10 equivalents, preferably 3 to 6 equivalents, of oxidant (alkaline salts of periodic acids, such as sodium periodic acid or potassium periodic acid), usually under 0 to 10°C, preferably under 0 to 4°C.

The shiff base may be formed by reaction of the aldehyde compound thus obtained and the lipid containing primary amino group (phospholipids such as phosphatidyl-ethanolamine). The reaction may be performed by mixing of two solutions, solution of said aldehyde compound and solution of said lipid, usually under temperature of 15 to 60°C. The aldehyde compound is dissolved in appropriate aqueous solvent such as water or phosphate buffer and said lipid is dissolved in appropriate organic solvent such as chloroform or methanol. During the reaction or after the completion of the reaction, appropriate reductant (alkaline salts of boron hydride, such as sodium boron hydride, sodium cyano boron hydride) may be added for reducing the shiff base.

When producing the lipid-bound glycosaminoglycan by this method, a spacer compound with two functional groups, one of which is primary amino group (for example, alkylenediamines such as ethylenediamine or amino acids such as lysine), may be used instead of the lipid containing primary amino group. The primary amino group of said spacer compound reacts with said aldehyde compound to form aminoalkyl bonding (-CH₂-NH-). The other functional group (for example, amino group) of said spacer compound (for example, monoacylglycerol dicarbonate ethers, such as monoacylglycerol succinate ether) may be reacted with a lipid containing a functional group capable of reacting with said another functional group (for example, carboxyl group).

### Lactonization of the reduced terminal of glycosaminoglycan

In this method, sugar residue existing at the reduced terminal of glycosaminoglycan is oxidized. The examples of such sugar residues are galactose residue, uronic acid residue and hexosamine residue. Then the pyranose ring existing at the reduced terminal of said glycosaminoglycan was specifically opened (cleaved) to form carboxyl group. The carboxyl group was subjected for the lactone formation reaction to form a lactone ring structure at the reduced terminal of glycosaminoglycan. The lactone ring thus produced was reacted with primary amino group of the lipid to form peptide bonding (-CO-NH-). This reaction results in formation of covalent bonding between the glycosaminoglycan and the lipid.

For the oxidation of sugar residue existing at the reduced terminal of glycosaminoglycan, the glycosaminoglycan was treated with about 2 to 20 equivalents, in preferably about 5 to 15 equivalents, of an oxidant (for example, iodine or bromide). The reaction may be performed in appropriate aqueous solvent (for example, water or phosphate buffer) usually under 0 to 40°C, in preferred form 15 to 30°C.

After oxidation reaction describe above, treatment with strong anion ion exchange resin and/or acid treatment may be performed. For examples of anion ion exchange resin available, Dowex 50 (Dow chemical Co., Ltd.) and Amberlite IR-120 (Organo Co., Ltd.) may be listed. The examples of acid available are inorganic acids such as hydrochloric acid or sulfuric acid and acid anhydrides of organic acids such as acetic acid, citric acid or succinic acid. Then the lactone compounds specifically lactonized at the reduced terminal of glycosaminoglycan may be produced.

The reaction of the lactonized compound thus obtained and the lipid containing primary amino group (phospholipids such as phosphatidyl ethanolamine) may be performed as follows. The lactonized compound is dissolved in appropriate aqueous solvent (water or phosphate buffer) and the lipid is dissolved in appropriate organic solvent (chloroform or methanol). Both solvents is mixed for the reaction performed under 5 to 80°C, preferably under 30 to 60°C.

Then, as the restricted oxidation of the reduced terminal of glycosaminoglycan described above, a spacer compound with two functional groups including primary amino group may be used instead of a lipid containing primary amino group. Said spacer compound may be reacted with said lactonized compound to form peptide bonding (-CO-NH-). The another functional group of the spacer compound may be reacted with the another functional group of the lipid (for example, carboxyl group).

It is to be understood that the method to produce lipid-bound glycosaminoglycan, wherein a lipid is conjugated to a glycosaminoglycan at its reduced terminal, is not to be limited to the methods described above. Any method can be adopted to produce the lipid-bound glycosaminoglycan so far as the lipid can bind to the glycosaminoglycan at its reduced terminal.

For the purpose to conjugate a lipid to a glycosaminoglycan through a binding site other than the reduced terminal, carboxyl group contained in uronic acid portion of the glycosaminoglycan is reacted with primary amino group of the lipid to form peptide bonding (-CO-NH-).

To perform the reaction described above, peptide bonding may be formed by the reaction using a condensing agent, such as 1-ethyl-3-(3-dimethylaminopropyl)carbodiimide or dichrolohexyl carbodiimide. Alternatively, carboxyl group of uronic acid may be reacted with an activating agent, such as N-hydroxy succinic imide, p-nitrophenol or N-hydroxybenzotriazole, under presence of said condensing agent to produce an activated ester.

### The activated ester may be reacted with the lipid to form peptide bonding.

In the reaction described above, uronic acid portion of the glycosaminoglycan may preferably be converted to a salt soluble in organic solvents. Then the reaction may be performed in a organic solvent. The salts soluble in organic solvents may preferably include but not limited to amine salts such as triethylamine or tributylamine. The organic solvents may preferably include but not limited to dimethylformamide, dimethylsulfoxide or pyridine.

The pharmaceutical accepted salts adopted for said lipid-bound glycosaminoglycan may preferably be alkaline metal salts such as potassium or sodium, alkaline-earth metal salts such as calcium or magnesium, amine salts such as trialkylamine or organic bases such as pyridine. The range of available salts is not to be limited by the examples described above. However, alkaline metal salts is particularly preferable and sodium salt is the most preferable.

The examples of lipid-bound glycosaminoglycans described above are dipalmytoyl-L-(a-phosphatidyl)-ethanolamine-bound hyaluronic acid, dipalmytoyl-L-(a-phosphatidyl)-ethanolamine-bound chondroitin sulfate, stearoyl-palmytoyl-phosphatidylserine-bound chondroitin sulfate and mono-stearoylglycerol succinate ester-bound chondroitin sulfate. The detailed structures and the methods of production of these lipid-bound glycosaminoglycans are described in Japanese patent laid-open publications "kokai" 80201/1992, 80202/1992 and 30979/1997.

The osteogenesis inducing medicine of this invention may be formulated with some appropriate pharmaceutical carrier or dilutor of liquid or solid form, such as excipient or stabilizer. The pharmaceutical medicine may be formulated to contain about 0.1 weight % to 90 weight % of lipid-bound glycosaminoglycan of this invention, with water, gelatin, sucrose, starch, magnesium stearate, talc, fat and oil originated from an animal or a plant, benzylalcohol, polyalkylenealcohol, petroleum oil resin, palm oil or lanolin.

The osteogenesis inducing medicine of this invention may be administrated with various growth factors to enhance curing of fracture or anosteoplasia, which is the aim of this invention. The growth factors may preferably be fibroblast growth factors (FGF: such as aFGF or bFGF), osteogenesis proteins (BMPs: such as TGF-β), vascular endothelial cell growth factors (VEGF), calcitrophic factors (such as estrogen, parathyroid hormone, 1,25(OH)₂D3 (activated vitamin) or dexamethasone) or transcriptional factors (such as cbfal). The growth factor may more preferably exhibit affinity with the glycosaminoglycan constituting the lipid-bound glycosaminoglycan, which is the effective ingredient of this invention. As the result of the affinity, the growth factor operates to enhance osteogenesis or growth of peripheral tissues caused by osteogenesis. The examples of such growth factors are FGF, BMPs and VEGF. The growth factor exhibiting affinity with the glycosaminoglycan described above may be administrated with the osteogenesis inducing medicine of this invention. As the result of co-administration to the target site, such as the site of fracture or anosteoplasia, the diffusion of said growth factor is delayed and the concentration of growth factor is maintained sufficient to be operative for a sustained period.

Prior to formation of lipid-bound glycosaminoglycan, the growth factor exhibiting affinity to the glycosaminoglycan may be mixed with the osteogenesis inducing medicine. Therefore, the growth factor may be conjugated to the glycosaminoglycan contained in the lipid-bound glycosaminoglycan, the effective ingredient of this invention. The growth factor-conjugated lipid-bound glycosaminoglycan described above, as the effective ingredient, may be administrated to the target organ. The growth factor-conjugated osteogenesis inducing medicine may be formulated to be a sustained release medicine. Therefore, it enables sustained release of said growth factor toward the target site or its peripheral tissues to achieve earlier cure of fracture or anosteoplasia.

The osteogenesis inducing medicine of this invention may be formulated to be a granular medicine, an ointment or a liquid medicine. The pharmaceutical medicine may be administrated directly by dissection of the target site and application of the medicine. In the case of a liquid medicine, direct administration may be achieved by injection without dissection. The materials known to be used for artificial bone or reinforcing of bone, such as hydroxyapatite, titanium rod or titanium mesh, may be soaked into the liquid solution of osteogenesis inducing medicine of this invention. The osteogenesis inducing medicine of this invention may be conjugated, adsorpted or impregnated on the surface of these materials. The materials may be shaped if necessary. Then said materials may be introduced to said target site. It results in indirect administration of the osteogenesis inducing medicine to the target site and enhancing of osteogenesis, the aim of this invention, may be achieved.

The dosage of osteogenesis inducing medicine of this invention should be selected according to the condition and body weight of the patient. In general, administration of 1 µg to 2000 mg of lipid-bound glycosaminoglycan per region is preferable.

The acute toxicity (LD₅₀) of osteogenesis inducing medicine of this invention was evaluated by intraperitoneal administration of mouse, on phosphatidyl-ethanolamine conjugated chondroitin sulfate and phosphatidyl-ethanolamine conjugated hyaluronic acid. The LD₅₀ values of both compounds were above 2000 mg/kg, ensuring sufficient safety at administration of these compounds to a living body.

This invention will be illustrated in detail by the following embodiment.

### EMBODIMENT 1

### Osteogenesis inducing activity of the lipid-bound glycosaminoglycan on cultured cell

### (1) Preparation of cultured cell derived from rat calvaria

Cultured cell derived from rat calvaria was isolated from Wister rats (obtained from sankyo-rabo) of three days old by following method. The rats were killed, decapitated and their calvarias were picked out. The tissue was flaked by a tweezer and suspended into a solution for enzyme treatment, containing 0.04% of EDTA, collagenase (crude type 1A : SIGMA : 0.1%) and trypsin (0.05%). The sample was treated in the enzyme solution for 30 min under temperature of 37°C. Then, the enzyme solution was harvested and cells extricated into the enzyme solution was harvested by centrifugation (RC-I). The enzyme solution was added again and digested at 37°C for 30 min. Cells extricated into the enzyme solution was designated as RC-II.

The RC-I and RC-II thus obtained were mixed and used for following experiments. The cell mixture of RC-I and RC-II was designated as RC-I,II cell in following description. The cell group was consisted of undifferentiated precursor cell of osteoblast, which exhibits few secretion of extracellular matrix. The RC-I and RC-II described above hardly expressed alkaline phosphatase (ALP), type 1 collagen (COL1) and osteocarcin (OC) gene. The genes described above are used for marker genes of osteoblast (T.R. Arnett et al, Methods in Bone Biology, 1988, Chapman & Hall).

### (2) Preparation of the lipid-bound glycosaminoglycan

The lipid-bound glycosaminoglycan was prepared according to the method described in embodiment 1 of Japanese patent laid-open publication "kokai" 80201/1992.

Five hundred mg of hyaluronic acid (sodium salt: derived from cock's comb, molecular weight 23,000, HA) was dissolved in 10 ml of water. Five ml of iodine solution dissolved in methanol was added and reacted for 6 hours at room temperature. Then 5 ml of 0.1 N KOH solution was added to the reaction solution and the color of iodine was diminished. Pellet was obtained by addition of ethanol saturated by potassium acetate. The pellet was filtrated, fully washed by ethanol and dried in vacuum. As the result, 423 mg of hyaluronic acid (sodium salt), having molecular weight of 23,000 with its reduced terminal oxidated, was obtained.

Four hundred mg hyaluronic acid described above was dissolved in 10 ml of water. The solution was filtrated through 50 ml of strong anion exchange resin (Dowex 50 (H⁺)) for one hour. Water solution, containing 390 mg of hyaluronic acid lactonized at its reduced terminal, was thus obtained.

The solution described above was neutralized in tri-n-butylamine and freeze-dried. Four hundred mg of tri-n-butylamine salt of hyaluronic acid lactonized at its reduced terminal was thus obtained.

Four hundred mg of tri-n-butylamine salt of hyaluronic acid with its reduced terminal lactonized as described above, was dissolved in 200 ml of dimethylformamide. Chloroform solution containing 27.6 mg of L-(α-phosphatidyl) ethanolamine dipalmytoyl was added to the solution described above and reacted for two hours at 70°C. Chloroform was dried and excess volume of sodium acetate solution was added to form sodium salt and then ethanol saturated by sodium acetate was added. The pellet thus obtained was filtrated and dissolved in 0.3M ammonium acetate solution. The solution was adsorpted to hydrophobic chromatography column (TSKgel phenyltoyopearl 650M, 400ml), washed by 0.3M ammonium acetate solution and the target compound was eluted by 30% methanol solution. The fraction eluted by 30% of methanol was concentrated under vacuum and purified by freeze-drying. Thirty-six mg of L-(a-phosphatidyl) ethanolamine dipalmytoyl conjugated hyaluronic acid (abbreviated to "HA-PE") was obtained as described above.

### (3) Estimation of osteogenesis inducing activity of the lipid-bound glycosaminoglycan

HA-PE produced as described above was dissolved in hank's solution at concentration of 5 µg/ml and 2 ml of hank's solution containing HA-PE was poured into polystyrene culture dish with diameter of 10 cm. The dish was coated by settling the dish for 16 hours at 4°C. The dish treated by hank's solution without HA-PE was used for control.

The RC-I,II cell suspension, prepared as described above, was poured on the dish at the density of 1x10⁴ cells/cm². The cell was cultured in α-minimum eagle medium (MEM : GIBCO) solution containing 1 % anti-biotics, anti-mycoplasma solution (containing 50 µg/ml ascorbic acid and 5mM β-glycerophosphate, GIBCO) and 10% fatal bovine serum (FBS : GIBCO).

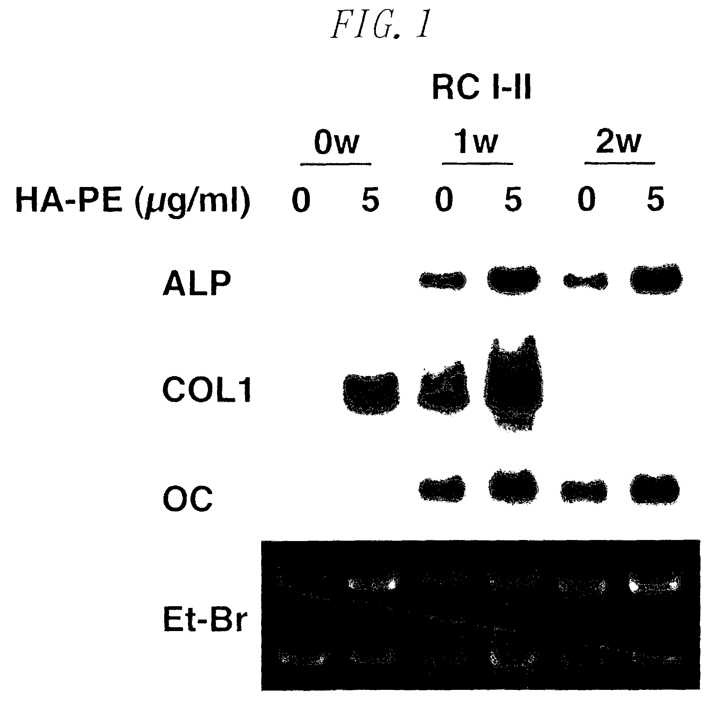

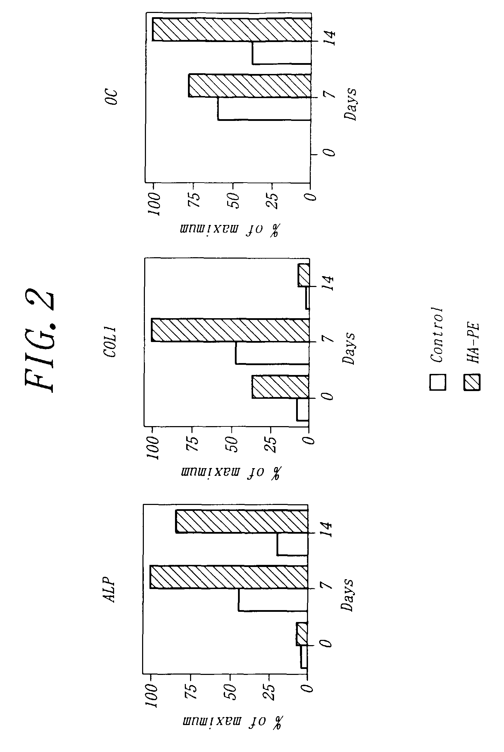

At the time just after cell reached to confluent, one week after confluent and two weeks after confluent, total RNA of the cell was extracted by guanidine-phenol-chloroform method. The expression of ALP, COL1 and OC genes was analyzed by northern blotting method. The expression of these genes are used as an indicator of osteoblast differentiation. The result was shown in Fig. 1. In Fig. 1, Et-Br indicates control sample obtained by staining of ribosomal RNA by ethydium bromide. Moreover, digitalized result calculated from northern blotting of Fig.1 by Quantity One program (TOYOBO) was shown in Fig.2 as a value standardized against the control described above.

As seen obviously from Fig.1 and Fig.2, especially digitalized result of Fig.2, RC-I,II cell cultured in HA-PE coated dish exhibited predominant increase on expression of ALP, CPL1 and OC genes. This result indicates significant induction of osteocyte differentiation caused by HA-PE.

This invention provided a novel medicine for induction of osteogenesis, which includes lipid-bound glycosaminoglycan as an effective ingredient. Therefore, diseases or injuries, bringing fracture or anosteoplasia, can be cured in a significant short period.

## Claims

1. An osteogenesis inducing medicine containing a lipid-bound glycosaminoglycan, composed of a glycosaminoglycan conjugated with a lipid, or a pharmacologycally accepted salt of the lipid-bound glycosaminoglycan as the effective ingredient of the medicine.

2. The osteogenesis inducing medicine according to claim 1, wherein the glycosaminoglycan is selected from the group consisting of hyaluronic acid, condroitin, chondroitin sulfate, chondroitin polysulfate, dermatan sulfate, heparin, keratan sulfate and keratan polysulfate.

3. The osteogenesis inducing medicine according to claim 1 or claim 2, wherein said lipid is a glycerolipid.

4. The osteogenesis inducing medicine according to claim 3, wherein said glycerolipid is a phospholipid.

5. The osteogenesis inducing medicine according to claim 4, wherein said phospholipid is phosphatidyl-ethanolamine.

6. The osteogenesis inducing medicine according to claim 1, wherein said lipid is hyaluronic acid and said lipid is phosphatidylethanolamine.

7. The osteogenesis inducing medicine according to either of claim 1 to claim 6, wherein said glycosaminoglycan has a reduced terminal conjugated with the lipid by covalent bonding.
